# EUROPEAN PATENT SPECIFICATION

(11) **EP 3 380 327 B1**
(45) Date of publication and mention of the grant of the patent: **08.07.2026**
(21) Application number: 15909026.5
(22) Date of filing: 25.11.2015
(51) Int. Cl.: B32B 27/32, A61F 13/15, B32B 7/05, B32B 5/02, B32B 5/06, B32B 5/08, B32B 5/26, B32B 7/02, B32B 7/04, B32B 37/04, B32B 37/06, D04H 1/4291, D04H 1/4334, D04H 1/435, D04H 1/4374, D04H 1/541, D04H 1/559, A61F 13/51, A61F 13/511, A61F 13/513

(54) **NONWOVEN AND ABSORBENT ARTICLES HAVING THE SAME**
VLIESSTOFF UND ABSORBIERENDE ARTIKEL DAMIT
NON TISSÉ ET ARTICLES ABSORBANTS LE COMPORTANT

(43) Date of publication of application: 03.10.2018
(73) Proprietor: The Procter & Gamble Company, Cincinnati, OH 45202 (US)
(72) Inventor: LIU, Xiaoxin, Beijing 101312 (CN); SONG, Limin, Beijing 101312 (CN); ISELE, Olaf Erik Alexander, Cincinnati, OH 45202 (US); WANG, Fancheng, Beijing 101312 (CN); TANG, Li, Beijing 101312 (CN); CAI, Jixiang, Xiamen Fujian 36110 (CN); ERDEM, Güeltekin, 65824 Schwalbach am Taunus (DE)
(74) Representative: P&G Patent Germany
(86) International application number: PCT/CN2015/095485
(87) International publication number: WO 2017/088114

(56) References cited:
- EP-A1- 0 539 703
- EP-A1- 0 539 703
- EP-A1- 1 369 518
- EP-A2- 1 227 181
- EP-A2- 1 323 400
- EP-A2- 1 323 400
- CN-A- 1 246 164
- JP-A- 2010 106 402
- JP-A- H05 176 953
- JP-A- H11 290 381
- US-A- 4 883 707
- US-A- 5 836 930
- US-A1- 2007 299 416
- US-B2- 7 179 247

## Description

### FIELD OF THE INVENTION

The present invention relates to nonwoven, a method for manufacturing the same, and also an absorbent article comprising the nonwoven.

### BACKGROUND OF THE INVENTION

Nonwovens including synthetic fibers formed from thermoplastic resin are widely used as sheets constituting absorbent articles such as sanitary napkins, infant disposable diapers, personal care disposable diapers, and the like.

Various nonwovens have been suggested for use as a component such as topsheets for absorbent articles from the standpoints of skin sensation, a feeling of dryness, comfort, absorption of expelled bodily fluids, and prevention of fluid flow-back. It is apparent that a cost-effective nonwoven while maintaining advantageous physical properties such as thickness, smoothness, cushioning and a desirable bulkiness would satisfy a long-feed need in the nonwoven textile art.

Thickness or loft of nonwoven is normally determined by the basis weight. Increasing the basis weight adds cost due to the use of more raw materials. It is desirable to have increased thickness in some applications where the nonwoven is used without increasing the basis weight. Desirable bulkiness of nonwoven is also normally determined by the basis weight. In some application it is desirable to have a fabric with increased desirable bulkiness in some application without increasing the basis weight.

Meanwhile, when a nonwoven is used as a component such as a topsheet of an absorbent article, the friction between the topsheet and the wearer's skin generates fuzz on the surface of the topsheet which often causes skin rash or uncomfortableness. Higher fuzz level results in greater skin irritation as well as a greater itching sensation to the skin. Therefore, decrease of fuzz generation during in use is advantageous.

US patent 4,883,707 discloses a high loft nonwoven fabric composites composed of a soft carded web layer comprising thermoplastic resin fibers having an average denier of 3 or less, and a lofty carded web layer comprising thermoplastic resin fibers having an average denier of 3 or greater. Japanese Unexamined Patent Publication No. 2006-233364 describes a nonwoven having smoothness and a soft comfortable feeling which comprises a first web layer and a second web layer, wherein at least the fiber included in the first web layer have a cross-section that is flat, and a major axis of said cross-section is oriented in a direction that is substantially parallel to a surface of the nonwoven. US 2015/173975A discloses a nonwoven having improved surface smoothness and tactile sensation, comprising a first fiber layer comprising a first eccentric core/sheath composite fiber composed of a sheath component comprising linear low density polyethylene, and a second fiber layer comprising a second eccentric core/sheath composite fiber composed of a sheath component comprising high density polyethylene.

There is a continuous need for a cost effective nonwoven with improved surface smoothness, an appropriate amount of cushioning and a desirable bulkiness and/or decrease of fuzz generation during usage. There is also a need for an absorbent article that provides improved tactile sensation, a feeling of dryness and comfort and /or limited fuzz generation.

### SUMMARY OF THE INVENTION

The present invention provides a nonwoven sheet as defined by claim 1; a method for manufacturing the nonwoven sheet, defined by claim 11; and an absorbent article comprising the nonwoven sheet, defined by claim 14.

### BRIEF DESCRIPTION OF THE DRAWINGS

FIG. 1 is a plan view of a set-up for fuzz measurement.
FIG. 2 is a grade scale for fuzz assessment in fuzz measurement.

### DETAILED DESCRIPTION OF THE INVENTION

The term "absorbent articles", as used herein, include disposable diapers, sanitary napkins, panty liners, incontinence pads, interlabial pads, breast-milk pads, sweat sheets, animal-use excreta handling articles, animal-use diapers, and the like.

The term "joined", as used herein, refers to the condition where a first member is attached, or connected, to a second member either directly or indirectly. Where the first member is attached, or connected, to an intermediate member which in turn is attached, or connected, to the second member, the first member and second member are joined indirectly.

A nonwoven of the present invention has at least two layers comprising a first web layer and a second web layer wherein the first web layer comprises a first core/sheath composite fiber having a fiber fineness no greater than 2.8 dtex (2.5 denier), and the second web layer comprises a first core/sheath composite fiber having a fiber fineness no greater than 2.8 dtex (2.5 denier) and a homopolymer having a fiber fineness greater than 3.3 dtex (3 denier). Advantageously, the nonwoven of the present invention have increased initial thickness and compressed thickness while maintaining surface smoothness. The nonwoven has limited fuzz generation in condition of friction with the skin. The advantageous properties of the nonwoven of the present invention, without being bound by theory, may be achieved employing at least two nonwoven layers and utilizing core/sheath composite fibers in selected ranges of fiber fineness, and employing a core/sheath composite fiber and a homopolymer in different fiber fineness in the second layer.

Without being bound by theory, sheath components of the core/sheath composite fibers comprising linear low density polyethylene may impart a luxurious tactile sensation such as surface softness and smoothness. The homopolymer in the second layer may mainly impart high bulkiness and cushioning to the nonwoven. The selected range of the homopolymer in the second layer may contribute to a decrease in fuzz level of the nonwoven even.

Hereinafter, the fiber constituting the nonwoven of the present invention, the configurations of the first and the second web layer, and a method for manufacturing the nonwoven, and an absorbent article having the sheet are described.

### First Core/Sheath Composite Fiber

The first core/sheath composite fiber in the present invention comprises a core component comprising a resin and a sheath component comprising a thermoplastic resin having a melting point of at least about 20°C lower than a melting point of the resin of the core component. The first core/sheath composite fiber has a fiber fineness no greater than about 2.8 dtex (2.5 denier), or no greater than about 2.2 dtex (2.0 denier). The nonwoven according to the present invention, without being bound by theory, may not have satisfactory smoothness of the surface of the nonwoven when the fiber fineness is greater than 2.8 dtex (2.5 denier). The first core/sheath composite fiber preferably has a fiber length less than about 100mm.

In the first core/sheath composite fiber, a composite ratio, that is, a volume ratio of core component/sheath component, is preferably from about 80/20 to about 30/70, more preferably from about 70/30 to about 35/65, and more preferably from about 60/40 to about 40/60. Without being bound by theory, in the first core/sheath composite fiber, the core component may principally contribute to a bulkiness (initial thickness) and a bulkiness recovery (compressible thickness) characteristics such as cushiony feel of the nonwoven, and the sheath component may principally contribute to strength and softness of the nonwoven. When the composite ratio is from about 80/20 to about 30/70, preferably about 70/30 to about 35/65, and more preferably from about 60/40 to about 40/60, both excellent strength and softness of the nonwoven and bulkiness recovery characteristics may be achieved. If the volume sheath component is increased, the strength of the resulting nonwoven may increase, but the nonwoven may harden and bulkiness recovery characteristics may be compromised. On the other hand, if the core component is excessive, there may be insufficient bonding points, the strength of the nonwoven may decrease and, as a result, bulkiness recovery characteristics may be negatively affected.

The first core/sheath composite fiber may have two-dimensional crimps and/or three-dimensional crimps. Herein, the term "two-dimensional crimp" can be understood mechanical crimping in which the peaks of the crimped fiber are sharply angled. Three-dimensional crimp may refer to crimp where the peaks are curved (wave shaped crimping) or spiral (spiral shaped crimping), crimp where both wave shaped crimping and spiral shaped crimping exist, or crimp where both mechanical crimp and at least one of wave and spiral shape crimps exist. In one embodiment, the first core/sheath composite fiber has two-dimensional crimps which is cost-effective compared to a composite fiber having three-dimensional crimps.

The first core/sheath composite fiber may be concentric or eccentric. In one embodiment, the first core/sheath composite fiber is a concentric fiber.

### Core Component

The core component comprises at least one resin, thermoplastic resin preferably. Resin for the core component preferably includes a polyolefin-based resin such as polypropylene, polymethylpentene, and the like; polyester resins such as polyethylene terephthalate, polybutylene terephthalate, polytrimethylene terephthalate, polyethylene naphthalate, polylactic acid, and copolymers thereof; polyamide-based resins such as nylon 66, nylon 12, nylon 6, and the like; acrylic resin; engineering plastics such as polycarbonate, polyacetal, polystyrene, cyclic polyolefin, and the like; mixtures thereof. For the perspectives of the uniformity of the nonwoven and nonwoven productivity, polyolefin resin, polyester and polyamide-based resin are more preferable. Examples of the polyester include polymers and copolymers such as polyethylene terephthalate, polybutylene terephthalate, polytrimethylene terephthalate, polyethylene naphthalate, polylactic acid. The core component may be selected from the group consisting of polyethylene terephthalate, polybutylene terephthalate, polypropylene, nylon, polyamide, and combinations thereof. Polyethylene terephthalate and polybutylene terephthalate are preferred, and polyethylene terephthalate are more preferred. Alternatively, the core component may comprise only polyester as a polymer component.

The core component may comprise additives other than resin, such as anti-static agents, pigments, matting agents, thermal stabilizers, light stabilizers, flame retardants, antimicrobial agents, lubricants, plasticizers, softeners, antioxidants, ultraviolet absorbers, crystal nucleating agents, and the like. These additives may be included in the core component at an amount that is not more than about 10 mass% of the core component.

### Sheath Component

The sheath component of the first core/sheath composite fiber comprises a thermoplastic resin having a melting point that is at least about 20°C lower than a melting point of the resin in the core component of the first core/sheath composite fiber.

The thermoplastic resin suitable for the sheath component may include resins described with respect to the core component above.

The sheath component may comprise additives other than resin, such as anti-static agents, pigments, matting agents, thermal stabilizers, light stabilizers, flame retardants, antimicrobial agents, lubricants, plasticizers, softeners, antioxidants, ultraviolet absorbers, crystal nucleating agents, and the like. These additives are preferably included in the sheath component at an amount that is not more than about 10 mass% of the entire sheath component.

### Second Core/Sheath Composite Fiber

The second core/sheath composite fiber in the present invention comprises a core component comprising a resin and a sheath component comprising a thermoplastic resin having a melting point of at least about 20°C lower than a melting point of the resin of the core component. The second core/sheath composite fiber has a fiber fineness no greater than about 2.8 dtex (2.5 denier) described below. When the fiber fineness is greater than 2.8 dtex (2.5 denier), smoothness of the surface of a nonwoven web made from the second core/sheath composite fiber may decline.

Descriptions with respect to the first core/sheath composite fiber in First Core/Sheath Composite Fiber above apply to the second core/sheath composite fiber.

### Homopolymer Fiber

The homopolymer in the present invention comprises at least one resin which doesn't melt or deform under the heat treatment during nonwoven manufacturing process.

Thermoplastic resin for the homopolymer fiber preferably includes a polyolefin-based resin such as polypropylene, polymethylpentene, and the like; polyester resins such as polyethylene terephthalate ( "PET" ) , polybutylene terephthalate, polytrimethylene terephthalate, polyethylene naphthalate, polylactic acid, and copolymers thereof; polyamide-based resins such as nylon 66, nylon 12, nylon 6, and the like; acrylic resin; engineering plastics such as polycarbonate, polyacetal, polystyrene, cyclic polyolefin, and the like; mixtures thereof. For the perspectives of the uniformity of the nonwoven and nonwoven productivity, polyolefin resin, polyester and polyamide-based resin are more preferable. Examples of the polyester include polymers and copolymers such as polyethylene terephthalate, polybutylene terephthalate, polytrimethylene terephthalate, polyethylene naphthalate, polylactic acid. The homopolymer may comprise at least one thermoplastic resin selected from the group consisting of polyethylene terephthalate, polybutylene terephthalate polypropylene, nylon, polyamide, and combinations thereof. Polyethylene terephthalate and polybutylene terephthalate are preferred, and polyethylene terephthalate are more preferred. Alternatively, the homopolymer may comprise only polyester as a polymer component. In one embodiment the homopolymer is PET.

The homopolymer fiber may comprise a shaped homopolymer fiber. Without being bound by theory, shaped fibers may be advantageous over round fibers to provide improved cushiony characteristics and compression resistance as shaped fibers have higher resilience at the same fiber denier due to having higher effective radius compared to round fibers. Shapes fibers also may introduce higher specific surface area which increases the capillary pressure of the second web layer containing shaped fibers which can lead to better drainage of the first web layer by the second fiber web layer comprising shape fibers. In one embodiment, the shaped homopolymer may be selected from the group consisting of bilobal shaped, trilobal shaped, quatro-lobal shaped, delta shaped, concave delta shaped, crescent shaped, oval shaped, star shaped, square shaped, U-shaped, **H-**shaped, C-shaped, V-shaped, diamond shaped fibers and any combinations thereof.

The homopoymer may comprise additives other than a resin illustrated with respect to the core/sheath composite fibers above.

Homopolymer fibers greater than about 3.3 dtex (3 denier), preferably greater than about 5.5 dtex (5 denier), without being bound by theory, may contribute to bulkiness characteristics.

The homopolymer fiber is preferably hydrophilic.

### First Web Layer

The first web layer in the nonwoven according to the present invention comprises the first core/sheath composite fiber having a fiber fineness no greater than about 2.8 dtex (2.5 denier). An average fiber fineness of fibers in the first web layer is preferable no greater than about 2.8 dtex (2.5 denier). In one embodiment, the first web layer contains only the first core/sheath composite fiber.

The first web layer may include other fibers in addition to the first core/sheath composite fiber. Examples of the other fibers include natural fibers such as cotton, silk, wool, hemp, pulp, and the like; reclaimed fiber such as rayon, cupra, and the like; and synthetic fibers such as acrylic-based, polyester-based, polyamide-based, polyolefin-based, and polyurethane-based fibers. One type or a plurality of types can be selected from these fibers, based on the application of the nonwoven.

### Second Web Layer

The second web layer in the nonwoven according to the present invention comprises the second core/sheath composite fiber and the homopolymer described under Second Core/Sheath Composite Fiber and Homopolymer above.

The second web layer may comprise the second core/sheath composite fiber which has a fiber fineness no greater than a fiber fineness of the first composite fiber in the first web layer. It may introduce a cappliary cascade in the nonwoven and be more effective in fluid transportion from the first web layer to the second web layer which can improve dryness and cleanness of an absorbent article when the nonwoven is used as a component of the absorbent article.

In one embodiment, the second core/sheath composite fiber and the homopolymer fiber have a fiber fineness difference no less than about 1.1 dtex (1 denier).

The second web layer comprises the homopolymer fiber in an amount not greater than about 25% by weight of the second web layer. The second web layer comprises the homopolymer fiber in an amount greater than 10% by weight, or optionally greater than 15% by weight of the second layer. Without being bound by theory, fuzz generation of the obtained nonwoven may be concerned when the homopolymer is more than 30%, and initial thickness and/or resilience of the obtained nonwoven may be concerned when the homopolymer is lower than 10%.

The second web layer may include other fibers in addition to the second core/sheath composite fiber and the homopolymer. Examples of the other fibers include natural fibers such as cotton, silk, wool, hemp, pulp, and the like; reclaimed fiber such as rayon, cupra, and the like; and synthetic fibers such as acrylic-based, polyester-based, polyamide-based, polyolefin-based, and polyurethane-based fibers. One type or a plurality of types can be selected from these fibers, based on the application of the nonwoven.

### Configuration of Nonwoven

The nonwoven of the present invention comprises a first web layer comprising a first core/sheath composite fiber, and a second web layer comprising a second core/sheath composite fiber and a homopolymer. At least a portion of the fibers is thermally bonded via the sheath components of the core/sheath composite fibers.

A basis weight of the nonwoven may be appropriately selected depending on the nonwoven application. For the nonwoven of the present invention as a topsheet of an absorbent article, the integral basis weight of the first web layer and the second web layer of the nonwoven is preferably from about 25g/m² to about 70g/m², more preferably about 35g/m² to about 65g/m². For the use of the nonwoven as a topsheet of an absorbent article, in one embodiment, the integral basis weight of the nonwoven is in the range of from about 30g/m² to about 70 g/m², and preferably from about 35g/m² to about 55g/m².

A basis weight of the first web layer and the second web layer, respectively, is preferably from about 5g/m² to about 50g/m², more preferably from about 10g/m² to about 40g/m², and even more preferably from about 14g/m² to about 35g/m². A ratio of a basis weight of the first web layer/the second web layer is preferably from about 80/20 to about 20/80 and more preferably from about 60/40 to about 50/50. If the basis weight of the first web layer is too small and/or the ratio of the basis weight of the first web layer to the basis weight of the second web layer is too small, softness and smoothness in the surface of the first web layer may decline. If the basis weight of the first web layer is too large and/or the ratio of the basis weight of the first web layer to the basis weight of the second web layer is too large, the bulkiness and the cushioning of the nonwoven may decline.

In one embodiment, the nonwoven may be constituted by only the first web layer and the second web layer. In another embodiment, the nonwoven comprises three layers in which the first web layer is layered on both faces of the second web layer. In another embodiment, the nonwoven may include at least one additional fiber layer in addition to the first and second web layers. The fiber for the additional web layer can be selected from natural fibers such as cotton, silk, wool, hemp, pulp, and the like; reclaimed fibers such as rayon, cupra, and the like; and synthetic fibers such as acrylic-based, polyester-based, polyamide-based, polyolefin-based, and polyurethane-based fibers. Such an additional fiber layer may comprise one or more types of fibers selected from these fibers.

The first web layer and the second web layer may comprise the same core/sheath composite fiber. In one embodiment, the first web layer comprises a first core/sheath composite fiber having a fiber fineness the same as or lower than a fiber fineness of a second core/sheath composite fiber in the second web layer. In another embodiment, the first web layer comprises a first core/sheath composite fiber having a fiber fineness higher than a fiber fineness of a second core/sheath composite fiber in the second web layer.

In one embodiment, the first web layer is less hydrophilic than the second layer in the nonwoven sheet of the present invention. In another embodiment, the first web layer is more hydrophilic than the second layer in the nonwoven sheet of the present invention.

In some embodiments, the nonwoven may have a fuzz grade not greater than 3, or not greater than 2.5, or not greater than 2 when measured according to Measurement of Fuzz Level described under the TEST MEHTODS.

The nonwoven of the present invention may comprise an opacifying agent from about 0.1 to about 6% by weight of dry weight of the nonwoven. An opacifying agent suitable for use includes titanium dioxide, clay, calcium carbonate, zinc oxide and diatomaceous silica.

### Nonwoven Manufacturing Process

The nonwoven may be manufactured via a process according to claim 11, a.o. comprising the steps of: forming a first fibrous web comprising a first core/sheath composite fiber having a fiber fineness no greater than about 2.5; forming a second fibrous web comprising a second core/sheath composite fiber having a fiber fineness no greater than about 2.8 dtex (2.5 denier) and a homopolymer fiber having a fiber fineness greater than about 3.3 dtex (3 denier) and less than or equal to 6.7 dtex (6 denier), wherein the homopolymer fiber is greater than 10% and not greater than about 25% by weight of the second fibrous web; forming a complex fibrous web by overlaying the first fibrous web on the second fibrous web; and subjecting the complex fibrous web to thermal treatment in order to thermal bond at least a portion of the first and the second core/sheath composite fibers via the sheath portions of the first and the second core/sheath composite fibers.

The first fibrous web and the second fibrous web may be carded webs such as parallel webs, semi-random webs, random webs, cross-webs, criss-cross webs, and the like, air-laid webs, wet-laid webs, and spunbond webs, and the like. The first and the second fibrous webs may be the same, or different.

The thermal treatment of a complex fibrous web can be conducted using any conventionally known thermal treatment method. Examples of preferable treating process include a thermal treatment apparatus such as a hot air through-type thermal treatment apparatus, a hot air blowing thermal treatment apparatus, a infrared thermal treatment apparatus, or the like. These thermal treatment apparatuses are typically provided with a conveying support for supporting and conveying a fibrous web. Thermal treatment may be performed under conditions such that the sheath components of the first and the second core/sheath composite fibers sufficiently melt and/or soften, and bond at a point of contact or intersection of the fibers, and such that crimps of the first and the second core/sheath composite fiber does not collapse. For example, the thermal treatment temperature may be from about 120°C to about 150°C, and preferably from about 128°C to about 145°C.

### Application of Nonwoven

The nonwoven of the present invention delivers a soft and smooth feel to the skin, has a bulky and feathery tactile sensation when the surface of the nonwoven is pressed against, and has an appropriate amount of cushioning and bulkiness recovery characteristics.

As such, the nonwoven of the present invention can be preferably used in applications in which the nonwoven is in contact with the skin, specifically applications in which the first web layer is the surface that is in contact with the skin. For example, the nonwoven of the present invention can be used in applications such as products that contact human or non-human animal skin, such as infant-use disposable diapers, adult-use disposable diapers, sanitary napkins, panty liners, incontinence pads, interlabial pads, breast-milk pads, sweat sheets, animal-use excreta handling articles, animal-use diapers, and similar various absorbent articles; face masks, base fabric of cooling/heating pads and similar cosmetic/medical-use patches, wound surface protection sheets, nonwoven bandages, hemorrhoid pads, warming devices that directly contact the skin (e.g. disposable hand warmers), base fabric of various animal-use patches, and similar skin covering sheets; makeup removal sheets, anti-perspirant sheets, bottom wipes and similar wipes for use on a person, various wiping sheets for use on animals, and the like. The nonwoven of the present invention is preferably used as a topsheet for an absorbent article in which the surface of first web layer is in contact with the skin.

### Absorbent Article

An absorbent article according to the present invention comprises a topsheet and a backsheet joined to the topsheet, wherein the topsheet comprises the nonwoven according to claim 1. It may further comprise an absorbent core disposed between the topsheet and the backsheet.

The absorbent articles of the present invention may be produced industrially by any suitable means. The different layers may thus be assembled using standard means such as embossing, thermal bonding, gluing or any combination thereof.

### Topsheet

Topsheet can catch body fluids and/or allow the fluid penetration inside the absorbent article. With the nonwoven according to the present invention, the first web layer is preferably, disposed on a side in contact with the skin.

### Backsheet

Any conventional liquid impervious backsheet materials commonly used for absorbent articles may be used as backsheet. In some embodiments, the backsheet may be impervious to malodorous gases generated by absorbed bodily discharges, so that the malodors do not escape. The backsheet may or may not be breathable.

### Absorbent core

It may be desirable that the absorbent article further comprises an absorbent core disposed between the topsheet and the backsheet. As used herein, the term "absorbent core" refers to a material or combination of materials suitable for absorbing, distributing, and storing fluids such as urine, blood, menses, and other body exudates. Any conventional materials for absorbent core suitable for absorbent articles may be used as absorbent core.

### TEST METHODS

### Measurement of Thickness of Nonwoven

Initial thickness of a nonwoven is measured using a tensile tester (Synergize 200, MTS System Corporation , USA) with a plunger to compress the material and a stage to carry the nonwoven.
1) Before running the measurement, zero the tester according to the manufacturer's instructions.
2) Measure the rough thickness of a sample using a ruler.
3) Set a tencile tester : Cross head speed: 0.02cm/sec. Pre-load = 2g.
4) Move manually the plunger down until it is close to but not touching the stage to put a space enough to place the sample between the plunger and the stage.
5) Set the program for the tencile tester to carry out steps (a)-(d) below automatically, and start the tencile tester to collect data according to the manufacturer's instructions. Between steps (c) and (d), place a sample (sample size 8cm x 8cm) on the stage to ensure the plunger position is aligned with the center of the sample.
   (a) Move the plunger down until it touches to the stage.
   (b) Move the plunger up to a platen separation position.
   (c) Wait until the sample is set under the plunger.
   (d) Move the plunger down until the load exceeds 35gf, then return it to the platen separation position. The platen separation position value is entered in the program before the tensile tester conducting steps (a)-(d).
6) Data analysis

Thickness at 2.0gf load defines T0 (mm), which indicates an initial thickness of a sample. Thickness at maximum pressure defines Tm (mm), which indicates a distance between the stage and the plunger at a defined maximum load. Difference between T0 and Tm, T0-Tm, defines compressible thickness (Tc).

### Measurement of Surface Smoothness

Surface smoothness is characterized by coefficient of friction ("COF") measured by a fabric touch tester (M293, SDL Atlas) according to the manufacturer's instructions.

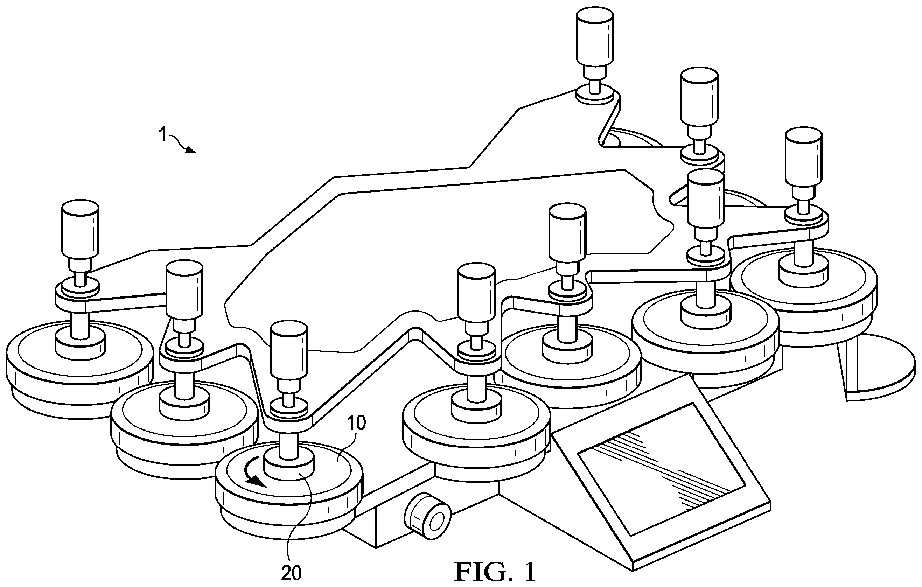

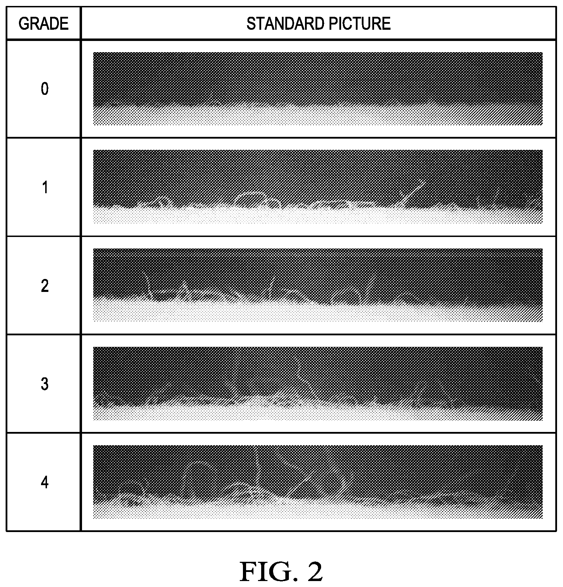

### Measurement of Fuzz Level (also, see FIGS. 1 and 2)

Fuzz of a nonwoven having a first side and a second side is measured using a martindale abrasion (YG(B)401G, Wenzhou DaRong) as below.
1) Cut a sample with 140mm in diameter ("large sample") and place it on a first sample holder (10) with standard felt so that a second side of the large sample faces a surface of the standard felt.
2) Cut the same sample with 38mm in diameter ("small sample") and place it on a second sample holder (20) with polyurethane foam so that a first side of the small sample faces a first side of the large sample on the standard felt (10).
3) Operate the martindale abrasion (1) so that the first side of the small sample on the second sample holder (20) rubs against the first side of the large sample on the first sample holder (10) under conditions below.
   pressure: 1.3Psi on the sample on the first sample holder (10)
   speed: 50 cycles per minute; and
   cycles number: 30 cycles

After the rubbing stops, evaluate a fuze level of the first surface of the large sample in 6 level scales with the density and the height of the fuzz by observing the large sample from 45 degree from the horizon according to grades in FIG. 2. A sample having a more severe fuzz level than Grade 4 is determined as Grade 5. The higher number indicates the more fuzz.

### EXAMPLES

### Examples 1-5

Various first web layers from various core/sheath composite fibers as indicated in Table 1were fabricated using a parallel carding machine. Various second web layers from various core/sheath composite fibers and homopolymers were fabricated as indicated in Table 1 using a parallel carding machine. A first fibrous web was overlaid on a second fibrous web as indicated in Table 1 and each overlaid web was subjected to thermal treatment at the temperatures 130 - 140°C. The thermal treatment was performed using a hot air through-type thermal treatment apparatus with a breathable conveyor belt. In the heat treatment, each of the complex webs was placed on the breathable conveyor belt of the thermal treatment apparatus so that the surface of the first web layer was in contact with the breathable conveyor belt. Thermal bonded nonwovens were obtained via the thermal treatment.

The obtained nonwovens were evaluated as described below.

Initial thickness and compressible thickness of nonwovens were measured according to Measurement of Thickness of Nonwoven described under TEST METHODS above and indicated in Table 1. Surface smoothness and fuzz level were measured according to Measurement of Surface Smoothness and Measurement of Fuzz Level under TEST METHODS above and indicated in Table 1.

**Table 1**

| | | Ex 1 | Ex 2 | Ex 3 | Ex 4 | Ex 5 |
|---|---|---|---|---|---|---|
| 1st layer | Composite fiber | PET/PE^{*1} | PET/PE^{*1} | PET/PE^{*1} | PET/PE^{*2} | PET/PE^{*2} |
| | Fiber fineness (denier) | 2.0 | 2.0 | 2.0 | 1.5 | 1.5 |
| | Basis weight (g/m²) | 15 | 15 | 20 | 15 | 15 |
| 2nd layer | Composite fiber | PET/PE^{*1} | PET/PE^{*1} | PET/PE^{*1} | PET/PE^{*2} | PET/PE^{*1} |
| | Composite fiber fineness (denier) | 2.0 | 2.0 | 2.0 | 1.5 | 2.0 |
| | Homopolymer | PET^{*3} | PET^{*3} | PET^{*3} | PET^{*3} | PET^{*3} |
| | Homopolymer fiber fineness (denier) | 6.0 | 6.0 | 6.0 | 6.0 | 6.0 |
| | Homopolymer amount (%) | 20 | 25 | 20 | 20 | 20 |
| | Basis weight (g/m²) | 25 | 25 | 20 | 25 | 25 |
| Nonwoven Properties | Initial thickness (mm) | 2.01 | 2.12 | 1.94 | 1.92 | 1.96 |
| | Compressible thickness (mm) | 1.39 | 1.45 | 1.20 | 1.22 | 1.30 |
| | Smoothness | 0.22 | 0.22 | 0.22 | 0.21 | 0.21 |
| | Fuzz | 2 | 3 | 2 | 2 | 2 |

| | | | | | | |
|---|---|---|---|---|---|---|
| *1: PET/PE: ETC214, JNC corporation. Japan *2: PET/PE: TA21, Jiangnan Fiber, China *3: PET: W40, Huvis, Korea | | | | | | |

### Comparative Examples 1-8

Various first fibrous webs from various core/sheath composite fibers were fabricated as indicated in Table 2 using a parallel carding machine. Various second fibrous webs from various core/sheath composite fibers and homopolymers were fabricated as indicated in Table 2 using a parallel carding machine. Fabrication of complex webs and thermal treatment were conducted according to the methods described under Examples 1-5 above. The obtained nonwovens were evaluated according to the methods described under Examples 1-5 above, and indicated in Table 2.

**Table 2**

| | | Com. Ex 1 | Com. Ex 2 | Com. Ex 3 | Com. Ex 4 | Com. Ex 5 | Com. Ex 6 |
|---|---|---|---|---|---|---|---|
| 1st Layer | Composite fiber | PET/PE^{*4} | PET/PE^{*5} | PET/PE^{*1} | PET/PE^{*1} | PET/PE^{*2} | PET/PE^{*1} |
| | Fiber fineness (denier) | 4 | 3 | 2 | 2 | 1.5 | 2 |
| | Basis weight (g/m²) | 15 | 15 | 15 | 15 | 15 | 2 |
| 2nd Layer | Composite fiber | PET/PE^{*1} | PET/PE^{*1} | PET/PE^{*1} | PET/PE^{*1} | PET/PE^{*2} | PET/PE^{*1} |
| | Composite fiber fineness (denier) | 2 | 2 | 2 | 2 | 1.5 | 2 |
| | Homopolymer | PET^{*3} | PET^{*3} | PET^{*3} | PET^{*3} | PET^{*6} (hollow) | PET/PE^{*7} |
| | Homopolymer fiber fineness (denier) | 6.0 | 6.0 | 6.0 | 6.0 | 7.0 | 6.0 |
| | Homopolymer amount (%) | 20 | 20 | 0 | 30 | 20 | 20 |
| | Basis weight (g/m²) | 25 | 25 | 25 | 25 | 25 | 25 |
| Nonwoven Properties | Initial thickness (mm) | 2.01 | 1.95 | 1.78 | 2.26 | 2.11 | 1.95 |
| | Compressible thickness (mm) | 1.10 | 1.07 | 1.13 | 1.57 | 1.38 | 1.12 |
| | Smoothness | 0.25 | 0.24 | 0.22 | 0.22 | 0.21 | 0.22 |
| | Fuzz | 2 | 2 | 1 | 5 | 5 | 1 |

| | | | | | | | |
|---|---|---|---|---|---|---|---|
| *1: PET/PE: ETC214, JNC corporation, Japan *2: PET/PE: TA21, Jiangnan Fiber, China *3: PET: W 40, Huvis, Korea *4: PET/PE: ETC244, Indorama, Thailand *5: PET/PE: ETC244, lndorama, Thailand *6: PET: HC_7D_64MM_AC, Toray *7: PET/PE: ETC214, JNC corporation | | | | | | | |

## Claims

1. A nonwoven sheet comprising
a first layer comprising a core/sheath first composite fiber having a fiber fineness no greater than 2.8 dtex (2.5 denier), wherein the first composite fiber comprises a core component comprising at least one thermoplastic resin selected from the group consisting of polyethylene terephthalate, polypropylene, nylon, polyamide, and combinations thereof, and a sheath component comprising a thermoplastic resin having a melting point of at least 20°C lower than a melting point of the core component;
a second layer comprising a core/sheath second composite fiber having a fiber fineness no greater than 2.8 dtex (2.5 denier), and a homopolymer fiber having a fiber fineness of greater than 3.3 dtex (3 denier) and less than or equal to 6.7 dtex (6 denier), wherein the second composite fiber comprises a core component comprising at least one thermoplastic resin selected from the group consisting of polyethylene terephthalate (PET) , polypropylene (PP) , nylon, polyamide, and combinations thereof, and a sheath component comprising a thermoplastic resin having a melting point of at least 20°C lower than a melting point of the core component,
wherein an average dtex (denier) of fibers in the first layer is no greater than 2.8 (2.5),
wherein the homopolymer fiber is greater than 10% and not greater than 25% by weight of the second layer.

2. The nonwoven sheet according to claim 1, wherein the second composite fiber and the homopolymer fiber have a fiber fineness difference no less than 3.3 dtex (3 denier).

3. The nonwoven sheet according to claim 1 or 2, wherein the first composite fiber and the second composite fiber have a fiber length less than 100mm.

4. The nonwoven sheet according to any of the preceding claims, wherein at least one of the first composite fiber and the second composite fiber has 2 dimensional crimp.

5. The nonwoven sheet according to any of the preceding claims, wherein the first composite fiber and the second composite fiber have a fiber fineness difference no greater than 1.1 dtex (1 denier).

6. The nonwoven sheet according to any of the preceding claims, wherein the first composite fiber has a fiber fineness no greater than a fiber fineness of the second composite fiber.

7. The nonwoven sheet according to any of the preceding claims, wherein the homopolymer fiber comprises a shaped homopolymer fiber.

8. The nonwoven sheet according to any of the preceding claims, wherein the first layer is less hydrophilic than the second layer.

9. The nonwoven sheet according to any of the preceding claims, wherein the ratio of a basis weight of the first fiber layer to a basis weight of the second layer is in the range of from 80/20 to 20/80.

10. The nonwoven sheet according to any of the preceding claims, wherein at least a portion of the first and the second core/sheath composite fibers is thermal bonded via the sheath components of the first and the second core/sheath composite fibers.

11. A method for manufacturing the nonwoven sheet according to claim 10, the method comprising the steps of:
forming a first fibrous web comprising a first core/sheath composite fiber having a fiber fineness no greater than 2.8 dtex (2.5 denier), wherein the first core/sheath composite fiber comprises a core component comprising at least one thermoplastic resin selected from the group consisting of polyethylene terephthalate (PET) , polypropylene (PP) , nylon, polyamide, and combinations thereof, and a sheath component comprising a thermoplastic resin having a melting point of at least 20°C lower than a melting point of the core component, and an average dtex (denier) of fibers in the first fibrous web is not greater than 2.8 (2.5),
forming a second fibrous web comprising a second core/sheath composite fiber having a fiber fineness no greater than 2.8 dtex (2.5 denier) and a homopolymer fiber having a fiber fineness greater than 3.3 dtex (3 denier) and less than or equal to 6.7 dtex (6 denier), wherein the second core/sheath composite fiber comprises a core component comprising at least one thermoplastic resin selected from the group consisting of polyethylene terephthalate, polypropylene, nylon, polyamide, and combinations thereof, and a sheath component comprising a thermoplastic resin having a melting point of at least 20°C lower than a melting point of the core component, and wherein the homopolymer fiber is greater than 10% and not greater than 25% by weight of the second fibrous web,
forming a complex fibrous web by overlaying the first fibrous web on the second fibrous web; and
subjecting the complex fibrous web to thermal treatment in order to thermal bond at least a portion of the first and the second core/sheath composite fibers via the sheath portions of the first and the second core/sheath composite fibers.

12. The method for manufacturing a nonwoven according to claim 11, wherein the thermal treatment is performed using a hot air through-type thermal treatment apparatus comprising a conveying support.

13. The method for manufacturing a nonwoven according to claim 12, wherein the first fibrous web is placed on the conveying support so that the first fiber layer contacts the conveying support during the thermal treatment.

14. An absorbent article comprising
a topsheet; and
a backsheet joined to the topsheet,
wherein the topsheet comprises the nonwoven according to claim 1.

15. The absorbent article according to claim 14, wherein the first fiber layer is positioned on a side in contact with the skin of a wearer.

## Patentansprüche

1. Vlieslage, umfassend
eine erste Schicht, umfassend eine erste Kern/Mantel-Verbundfaser, die eine Faserfeinheit von nicht mehr als 2,8 dtex (2,5 Denier) aufweist, wobei die erste Verbundfaser eine Kernkomponente umfasst, umfassend wenigstens ein thermoplastisches Harz, das aus der Gruppe ausgewählt ist, bestehend aus Polyethylenterephthalat, Polypropylen, Nylon, Polyamid und Kombinationen davon, und eine Mantelkomponente, umfassend ein thermoplastisches Harz, das einen Schmelzpunkt von wenigstens 20 °C niedriger als ein Schmelzpunkt der Kernkomponente aufweist;
eine zweite Schicht, umfassend eine zweite Kern/Mantel-Verbundfaser, die eine Faserfeinheit von nicht mehr als 2,8 dtex (2,5 Denier) aufweist, und eine Homopolymerfaser, die eine Faserfeinheit von mehr als 3,3 dtex (3 Denier) und gleich oder weniger als 6,7 dtex (6 Denier) aufweist, wobei die zweite Verbundfaser eine Kernkomponente umfasst, umfassend wenigstens ein thermoplastisches Harz, das aus der Gruppe ausgewählt ist, bestehend aus Polyethylenterephthalat (PET), Polypropylen (PP), Nylon, Polyamid und Kombinationen davon, und eine Mantelkomponente, umfassend ein thermoplastisches Harz, das einen Schmelzpunkt von wenigstens 20 °C niedriger als ein Schmelzpunkt der Kernkomponente aufweist,
wobei ein durchschnittliches dtex (Denier) von Fasern in der ersten Schicht nicht mehr als 2,8 (2,5) beträgt,
wobei die Homopolymerfaser mehr als 10 Gew.-% und nicht mehr als 25 Gew.-% der zweiten Schicht beträgt.

2. Vlieslage nach Anspruch 1, wobei die zweite Verbundfaser und die Homopolymerfaser einen Faserfeinheitsunterschied von nicht weniger als 3,3 dtex (3 Denier) aufweisen.

3. Vlieslage nach Anspruch 1 oder 2, wobei die erste Verbundfaser und die zweite Verbundfaser eine Faserlänge von weniger als 100 mm aufweisen.

4. Vlieslage nach einem der vorstehenden Ansprüche, wobei wenigstens eine der ersten Verbundfaser und der zweiten Verbundfaser eine 2-dimensionale Kräuselung aufweist.

5. Vlieslage nach einem der vorstehenden Ansprüche, wobei die erste Verbundfaser und die zweite Verbundfaser einen Faserfeinheitsunterschied von nicht mehr als 1,1 dtex (1 Denier) aufweisen.

6. Vlieslage nach einem der vorstehenden Ansprüche, wobei die erste Verbundfaser eine Faserfeinheit aufweist, die nicht größer als eine Faserfeinheit der zweiten Verbundfaser ist.

7. Vlieslage nach einem der vorstehenden Ansprüche, wobei die Homopolymerfaser eine geformte Homopolymerfaser umfasst.

8. Vlieslage nach einem der vorstehenden Ansprüche, wobei die erste Schicht weniger hydrophil als die zweite Schicht ist.

9. Vlieslage nach einem der vorstehenden Ansprüche, wobei das Verhältnis eines Flächengewichtes der ersten Faserschicht zu einem Flächengewicht der zweiten Schicht im Bereich von 80/20 bis 20/80 liegt.

10. Vlieslage nach einem der vorstehenden Ansprüche, wobei wenigstens ein Anteil der ersten und der zweiten Kern/Mantel-Verbundfaser über die Mantelkomponenten der ersten und der zweiten Kern/Mantel-Verbundfaser thermisch verklebt ist.

11. Verfahren zum Herstellen der Vlieslage nach Anspruch 10, das Verfahren umfassend die Schritte:
Ausbilden einer ersten Faserbahn, umfassend eine erste Kern/Mantel-Verbundfaser, die eine Faserfeinheit von nicht mehr als 2,8 dtex (2,5 Denier) aufweist, wobei die erste Kern/Mantel-Verbundfaser eine Kernkomponente umfasst, umfassend wenigstens ein thermoplastisches Harz, das aus der Gruppe ausgewählt ist, bestehend aus Polyethylenterephthalat (PET), Polypropylen (PP), Nylon, Polyamid und Kombinationen davon, und eine Mantelkomponente, umfassend ein thermoplastisches Harz, das einen Schmelzpunkt von wenigstens 20 °C niedriger als ein Schmelzpunkt der Kernkomponente aufweist, und ein durchschnittliches dtex (Denier) von Fasern in der ersten Faserbahn nicht mehr als 2,8 (2,5) beträgt,
Ausbilden einer zweiten Faserbahn, umfassend eine zweite Kern/Mantel-Verbundfaser, die eine Faserfeinheit von nicht mehr als 2,8 dtex (2,5 Denier) aufweist, und eine Homopolymerfaser, die eine Faserfeinheit von mehr als 3,3 dtex (3 Denier) und weniger oder gleich 6,7 dtex (6 Denier) aufweist, wobei die zweite Kern/Mantel-Verbundfaser eine Kernkomponente umfasst, umfassend wenigstens ein thermoplastisches Harz, das aus der Gruppe ausgewählt ist, bestehend aus Polyethylenterephthalat, Polypropylen, Nylon, Polyamid und Kombinationen davon, und eine Mantelkomponente, umfassend ein thermoplastisches Harz, das einen Schmelzpunkt von wenigstens 20 °C niedriger als ein Schmelzpunkt der Kernkomponente aufweist, und wobei die Homopolymerfaser mehr als 10 % und nicht mehr als 25 Gew.-% der zweiten Faserbahn beträgt,
Ausbilden einer komplexen Faserbahn durch Überlagern der ersten Faserbahn auf die zweite Faserbahn; und
Aussetzen der komplexen Faserbahn einer Wärmebehandlung, um wenigstens einen Teil der ersten und der zweiten Kern/Mantel-Verbundfaser über die Mantelanteile der ersten und der zweiten Kern/Mantel-Verbundfaser thermisch zu verkleben.

12. Verfahren zum Herstellen eines Vlieses nach Anspruch 11, wobei die Wärmebehandlung unter Verwendung eines Heißluftdurchströmungswärmebehandlungsapparats durchgeführt wird, umfassend eine Förderstütze.

13. Verfahren zum Herstellen eines Vlieses nach Anspruch 12, wobei die erste Faserbahn auf der Förderstütze platziert wird, so dass die erste Faserschicht mit der Förderstütze während der Wärmebehandlung in Kontakt gebracht wird.

14. Absorptionsartikel, umfassend
eine Oberschicht; und
eine Unterschicht, die mit der Oberschicht verbunden ist,
wobei die Oberschicht das Vlies nach Anspruch 1 umfasst.

15. Absorptionsartikel nach Anspruch 14, wobei die erste Faserschicht auf einer Seite angeordnet ist, die in Kontakt mit der Haut eines Trägers steht.

## Revendications

1. Feuille non tissée comprenant
une première couche comprenant une première fibre composite à noyau/gaine ayant une finesse de fibre ne dépassant pas 2,8 dtex (2,5 deniers), dans laquelle la première fibre composite comprend un composant central comprenant au moins une résine thermoplastique choisie dans le groupe constitué de téréphtalate de polyéthylène, polypropylène, nylon, polyamide, et combinaisons de ceux-ci, et un composant de gaine comprenant un résine thermoplastique ayant un point de fusion d'au moins 20 °C inférieur à un point de fusion du composant central ;
une seconde couche comprenant une seconde fibre composite à noyau/gaine ayant une finesse de fibre ne dépassant pas 2,8 dtex (2,5 deniers), et une fibre homopolymère ayant une finesse de fibre supérieure à 3,3 dtex (3 deniers) et inférieure ou égale à 6,7 dtex (6 deniers), dans laquelle la seconde fibre composite comprend un composant central comprenant au moins une résine thermoplastique choisie dans le groupe constitué de téréphtalate de polyéthylène (PET), polypropylène (PP), nylon, polyamide, et leurs combinaisons, et un composant de gaine comprenant une résine thermoplastique ayant un point de fusion d'au moins 20 °C inférieur à un point de fusion du composant central,
dans laquelle un dtex (denier) moyen de fibres dans la première couche n'est pas supérieur à 2,8 (2,5),
dans laquelle la fibre homopolymère est supérieure à 10 % et n'est pas supérieure à 25 % en poids de la seconde couche.

2. Feuille non tissée selon la revendication 1, dans laquelle la seconde fibre composite et la fibre homopolymère ont une différence de finesse de fibre d'au moins 3,3 dtex (3 deniers).

3. Feuille non tissée selon la revendication 1 ou 2, dans laquelle la première fibre composite et la seconde fibre composite ont une longueur de fibre inférieure à 100 mm.

4. Feuille non tissée selon l'une quelconque des revendications précédentes, dans laquelle au moins l'une parmi la première fibre composite et la seconde fibre composite a un sertissage bidimensionnel.

5. Feuille non tissée selon l'une quelconque des revendications précédentes, dans laquelle la première fibre composite et la seconde fibre composite ont une différence de finesse de fibre n'excédant pas 1,1 dtex (1 denier).

6. Feuille non tissée selon l'une quelconque des revendications précédentes, dans laquelle la première fibre composite a une finesse de fibre n'étant pas supérieure à une finesse de fibre de la seconde fibre composite.

7. Feuille non tissée selon l'une quelconque des revendications précédentes, dans laquelle la fibre homopolymère comprend une fibre homopolymère façonnée.

8. Feuille non tissée selon l'une quelconque des revendications précédentes, dans laquelle la première couche est moins hydrophile que la seconde couche.

9. Feuille non tissée selon l'une quelconque des revendications précédentes, dans laquelle le rapport d'une masse surfacique de la première couche de fibres à une masse surfacique de la seconde couche se situe dans la plage allant de 80/20 à 20/80.

10. Feuille non tissée selon l'une quelconque des revendications précédentes, dans laquelle au moins une partie des première et seconde fibres composites à noyau/gaine est liée thermiquement par l'intermédiaire des composants de gaine des première et seconde fibres composites à noyau/gaine.

11. Procédé de fabrication d'une feuille non tissée selon la revendication 10, le procédé comprenant l'étape consistant à :
la formation d'une première bande fibreuse comprenant une première fibre composite à noyau/gaine ayant une finesse de fibre n'excédant pas 2,8 dtex (2,5 deniers), dans lequel la première fibre composite à noyau/gaine comprend un composant central comprenant au moins une résine thermoplastique choisie dans le groupe constitué de téréphtalate de polyéthylène (PET), polypropylène (PP), nylon, polyamide, et des combinaisons de ceux-ci, et un composant de gaine comprenant une résine thermoplastique ayant un point de fusion d'au moins 20 °C inférieur à un point de fusion du composant central, et une moyenne dtex (denier) de fibres dans la première bande fibreuse n'est pas supérieur à 2,8 (2,5),
la formation d'une seconde bande fibreuse comprenant une seconde fibre composite à noyau/gaine ayant une finesse de fibre ne dépassant pas 2,8 dtex (2,5 deniers) et une fibre homopolymère ayant une finesse de fibre supérieure à 3,3 dtex (3 deniers) et inférieure ou égale à 6,7 dtex (6 deniers), dans lequel la seconde fibre composite à noyau/gaine comprend un composant central comprenant au moins une résine thermoplastique choisie dans le groupe constitué de téréphtalate de polyéthylène, polypropylène, nylon, polyamide, et des combinaisons de ceux-ci, et un composant de gaine comprenant une résine thermoplastique ayant un point de fusion d'au moins 20 °C inférieur à un point de fusion du composant central, et dans lequel la fibre homopolymère est supérieure à 10 % et n'est pas supérieure à 25 % en poids de la seconde bande fibreuse,
la formation d'une bande fibreuse complexe en superposant la première bande fibreuse sur la seconde bande fibreuse ; et
la soumission de la bande fibreuse complexe à un traitement thermique afin de lier thermiquement au moins une partie des première et seconde fibres composites à noyau/gaine par l'intermédiaire des parties de gaine des première et seconde fibres composites à noyau/gaine.

12. Procédé de fabrication d'un non-tissé selon la revendication 11, dans lequel le traitement thermique est mis en œuvre à l'aide d'un appareil de traitement thermique de type air chaud comprenant un support de transport.

13. Procédé de fabrication d'un non-tissé selon la revendication 12, dans lequel la première bande fibreuse est placée sur le support de transport de sorte que la première couche de fibres entre en contact avec le support de transport pendant le traitement thermique.

14. Article absorbant comprenant
une feuille supérieure ; et
une feuille de fond jointe à la feuille de dessus ;
dans lequel la feuille de dessus comprend le non-tissé selon la revendication 1.

15. Article absorbant selon la revendication 14, dans lequel la première couche de fibres est positionnée sur un côté en contact avec la peau d'un porteur.
